# EUROPEAN PATENT APPLICATION

(11) **EP 3 309 255 A1**
(43) Date of publication of application: **18.04.2018**
(21) Application number: 16761120.1
(22) Date of filing: 14.03.2016
(51) Int. Cl.: C12N 15/82, A01H 5/00

(54) **METHOD FOR INCREASING ABILITY OF PLANT TO RESIST INVADING DNA VIRUS**

(30) Priority: 12.03.2015 CN 201510107492
(71) Applicant: Institute Of Genetics And Developmental Biology, Chinese Academy Of Sciences, Beijing 100101 (CN)
(72) Inventor: GAO, Caixia, Beijing 100101 (CN); JI, Xiang, Beijing 100101 (CN); ZHANG, Huawei, Beijing 100101 (CN)
(74) Representative: Grünecker Patent- und Rechtsanwälte PartG mbB
(86) International application number: PCT/CN2016/076246
(87) International publication number: WO 2016/141893

(57) **Abstract**

The present invention discloses a method for improving the ability to resist against an intrusive DNA virus of a plant. The present invention provides a method for making a plant with improved ability to resist against a DNA virus, specifically can comprising the following steps: 1) selecting a sequence comply with 5'-N_{X}-NGG-3' or 5'-CCN-N_{X}-3' from the genomic sequence of the DNA virus as the target sequence; designing a DNA sequence reverse-complementary to said target sequence; 2) constructing said DNA sequence to a vector for expressing CRISPR/Cas9 nuclease so as to obtain a recombinant vector capable of transcribing a guide RNA and expressing a Cas9 protein; the crRNA in said guide RNA contains a RNA fragment transcribed from said DNA fragment; 3) introducing said recombinant vector into a recipient plant so as to obtain a plant with improved ability to resist against said DNA virus. The method of the invention merely depends on the genomic sequence of the virus, without the need of knowing specific functions of the viral genes. Therefore, the method can be widely used for resisting against various known double-stranded DNA virus or single-stranded virus with double-stranded DNA as an intermediate.

Sequence listing

<110>  Institute of Genetics and Developmental Biology, Chinese Academy of
Sciences

<120>  Method for improving ability to resist against intrusive DNA viruses
of plant

<130>  GNCLN150347
<160>  17
<170>  PatentIn version 3.5

<210>  1
<211>  23
<212>  DNA
<213>  Artificial
<220>
<223>
<400>  1
tcattggatt atcatagaca agg                                              23

<210>  2
<211>  23
<212>  DNA
<213>  Artificial
<220>
<223>
<400>  2
attatcatag acaaggatgt tgg                                              23

<210>  3
<211>  23
<212>  DNA
<213>  Artificial
<220>
<223>
<400>  3
cctactaagt tatcgagtat att                                              23

<210>  4
<211>  23
<212>  DNA
<213>  Artificial
<220>
<223>
<400>  4
tgatattccc gataacggtc agg                                              23

<210>  5
<211>  23
<212>  DNA
<213>  Artificial
<220>
<223>
<400>  5
ccgtctactt atcgtattcg aag                                              23

<210>  6
<211>  23
<212>  DNA
<213>  Artificial
<220>
<223>

<400>  6
ttcgaagaga tatgaacgag agg                                              23

<210>  7
<211>  23
<212>  DNA
<213>  Artificial
<220>
<223>
<400>  7
ggtttattgt gaagaagaaa tgg                                              23

<210>  8
<211>  23
<212>  DNA
<213>  Artificial
<220>
<223>
<400>  8
agaactcatt tgatgtctac tgg                                              23

<210>  9
<211>  23
<212>  DNA
<213>  Artificial
<220>
<223>
<400>  9
tggtactgga tatggaggga agg                                              23

<210>  10
<211>  23
<212>  DNA
<213>  Artificial
<220>
<223>
<400>  10
tggagggaag gagacttaca agg                                              23

<210>  11
<211>  23
<212>  DNA
<213>  Artificial
<220>
<223>
<400>  11
ccttcaatgc caaattacaa gaa                                              23

<210>  12
<211>  23
<212>  DNA
<213>  Artificial
<220>
<223>
<400>  12
ccgatgaata tcaatgtccg caa                                              23

<210>  13
<211>  23
<212>  DNA
<213>  Artificial
<220>
<223>

<400> 13
atctgaacat gaggactatt tgg 23

<210> 14
<211> 23
<212> DNA
<213> Artificial
<220>
<223>
<400> 14
actatttgga aagacaccgg tgg 23

<210> 15
<211> 23
<212> DNA
<213> Artificial
<220>
<223>
<400> 15
tgggaagtat gaagacgtga agg 23

<210> 16
<211> 2391
<212> DNA
<213> Artificial
<220>
<223>
<400> 16
aattccatac ccgtggactt ggtaccgaat ccattgtacc tcaaactcca ggacttcttc 60
cgttccgggc cagtttatca actgaaagtc cagataagat tcaaccacaa cctccgcaaa 120
tacttgaatc ttcacaagtg ctggatagat ttgacgatca ctggatcgca caggacatta 180
actggggacc gttttttgaa agtcttgaaa aagagactag agatatactt ggataattta 240
ggtttaattt gtattaataa tgtaattaga ggtttaaatc atgtcctgta tgaagaattt 300
aattttgtat ctagtgtaat tcagaaccag agtgttgcaa tgaaattgta ttaaaaaaat 360
aatattttta ttaataaaaa taacatctac aattgccaaa caaagtggca tacatattag 420
tattatccgt attatcatta acaacaacat agagtaaagc attctccttc acgtcttcat 480
acttcccacc accggtgtct ttccaaatag tcctcatgtt cagattgcgg acattgatat 540
tcatcggttt cttgtaattt ggcattgaag gagccttgta agtctccttc cctccatatc 600
cagtaccagt agacatcaaa tgagttctcc atttcttctt cacaataaac ctctcgttca 660
tatctcttcg aatacgataa gtagacggca tagcctgacc gttatcggga atatcaaata 720
tactcgataa cttagtagga aacactgacc caacatcctt gtctatgata atccaatgat 780
attgggtcat ataattagga gtctcccaga acggagaact ccccataaga gccatatcaa 840
acttcaaatg cttcgtcaca gtaacgttct tcgctctctg actatcacca ataccctgga 900
cataattatt caacatagta atcataccat tattactaat ggtagatcct ataccaccag 960
cacccaacat atcatcatac atctttaact tcagagtcct gttttttgtc acaggtctcc 1020
tccattggta tttcctcgaa atcgtagtag tcctcctctt cttcggccac gcactttgag 1080
gattcacctt tcttttctgg gacattgtat acgtatttct tgtatatttc ctcatacaac 1140
gaacacttcc tatgaaaatt aaccttacgc ctcagtagct tcttcacttc cttctggaat 1200
tgaagaaaga ccgcgccttc ttctagggtc agagcctcta tctcttgatg gatacctagt 1260
atacaccact tgttgtacct taagaaacaa ctggtcgata ctgctagaaa ggctggataa 1320
ttgtctggaa attgatccac tctgaaaggt cccataaaag ttcgtacctg attgcaggag 1380
aatactgaag ataaatagca aaaataacca gtctggtaga cagaccatca tttataagta 1440
catatacatg taaaaataac gtatttaaac agaaataatt cgaaacttgc ttagcaagtt 1500
tcttgtgggg accacaatga tctactttta actttatttc aaacagtatg tcggctactt 1560
ttttgactaa gtcaaaaaaa ggggcgggaa tgaaaacttc ttcaggaagt ttcccgccca 1620
aatccatcac atcaacatct ttagctttcc acgtggcata ttttcattgg gccacgtatt 1680
ttttgggggc catccggtaa tattaatgcg gatggcccct tggtggtatt taagagcacc 1740
caaagcaccc aattgatagg ggatttgaag agagcccgat tcaatatgcc tttttacaaa 1800
aaagccaaaa attttttcct tacataccct caatgttcag taaccaaaga agacgcctta 1860
gaacagctcc tcgctataaa tacaccttcg aataaaaaat atattcgcat ctgcagagaa 1920
ttacatgaaa atggggaacc acatctgcat gcccttattc aattcgaagg aaaagtccag 1980
atccgtaatg cccgttactt cgatctgcaa catcgaagta ccagcaaaca attccactgc 2040
aatattcagg gagctaaatc cagttccgac gtcaagtcct acgtctcaaa ggacggagat 2100

cacatcgact ggggtgaatt tcaggtcgat ggaagatctg cacgcggagg tcaacagacg 2160
gctaatgatg ctgcagcaga ggcattaaat gcaggtaatg cattagaagc tctgcagata 2220
ataagggaga aactcccaga aaaatatatt tttcagtatc acaacctcaa acctaactta 2280
gaggctattt ttcttcctcc tccagatctt tatcaaccac cttttcctct ttcttctttc 2340
actagagttc cagaaattat tcaagagtgg gccgattctt attttggttt g 2391

<210> 17
<211> 2927
<212> DNA
<213> Artificial
<220>
<223>
<400> 17
aattccatac ccgtggactt ggtaccgaat ccattgtacc tcaaactcca ggacttcttc 60
cgttccgggc cagtttatca actgaaagtc cagataagat tcaaccacaa cctccgcaaa 120
tacttgaatc ttcacaagtg ctggatagat ttgacgatca ctggatcgca caggacatta 180
actggggacc gttttttgaa agtcttgaaa aagagactag agatatactt ggataattta 240
ggtttaattt gtattaataa tgtaattaga ggtttaaatc atgtcctgta tgaagaattt 300
aattttgtat ctagtgtaat tcagaaccag agtgttgcaa tgaaattgta ttaaaaaaat 360
aatattttta ttaataaaaa taacatctac aattgccaaa caaagtggca tacatattag 420
tattatccgt attatcatta acaacaacat agagtaaagc attctccttc acgtcttcat 480
acttcccacc accggtgtct ttccaaatag tcctcatgtt cagattgcgg acattgatat 540
tcatcggttt cttgtaattt ggcattgaag gagccttgta agtctccttc cctccatatc 600
cagtaccagt agacatcaaa tgagttctcc atttcttctt cacaataaac ctctcgttca 660
tatctcttcg aatacgataa gtagacggca tagcctgacc gttatcggga atatcaaata 720
tactcgataa cttagtagga aacactgacc caacatcctt gtctatgata atccaatgat 780
attgggtcat ataattagga gtctcccaga acggagaact ccccataaga gccatatcaa 840
acttcaaatg cttcgtcaca gtaacgttct tcgctctctg actatcacca ataccctgga 900
cataattatt caacatagta atcataccat tattactaat ggtagatcct ataccaccag 960
cacccaacat atcatcatac atctttaact tcagagtcct gttttttgtc acaggtctcc 1020
tccattggta tttcctcgaa atcgtagtag tcctcctctt cttcggccac gcactttgag 1080
gattcacctt tcttttctgg gacattgtat acgtatttct tgtatatttc ctcatacaac 1140
gaacacttcc tatgaaaatt aaccttacgc ctcagtagct tcttcacttc cttctggaat 1200
tgaagaaaga ccgcgccttc ttctagggtc agagcctcta tctcttgatg gatacctagt 1260
atacaccact tgttgtacct taagaaacaa ctggtcgata ctgctagaaa ggctggataa 1320
ttgtctggaa attgatccac tctgaaaggt cccataaaag ttcgtacctg attgcaggag 1380
aatactgaag ataaatagca aaaataacca gtctggtaga cagaccatca tttataagta 1440
catatacatg taaaaataac gtatttaaac agaaataatt cgaaacttgc ttagcaagtt 1500
tcttgtgggg accacaatga tctactttta actttatttc aaacagtatg tcggctactt 1560
ttttgactaa gtcaaaaaaa ggggcgggaa tgaaaacttc ttcaggaagt ttcccgccca 1620
aatccatcac atcaacatct ttagctttcc acgtggcata ttttcattgg gccacgtatt 1680
ttttgggggc catccggtaa tattaatgcg gatggcccct tggtggtatt taagagcacc 1740
caaagcaccc aattgatagg ggatttgaag agagcccgat tcaatatgcc tttttacaaa 1800
aaagccaaaa attttttcct tacataccct caatgttcag taaccaaaga agacgcctta 1860
gaacagctcc tcgctataaa tacaccttcg aataaaaaat atattcgcat ctgcagagaa 1920
ttacatgaaa atggggaacc acatctgcat gcccttattc aattcgaagg aaaagtccag 1980
atccgtaatg cccgttactt cgatctgcaa catcgaagta ccagcaaaca attccactgc 2040
aatattcagg gagctaaatc cagttccgac gtcaagtcct acgtctcaaa ggacggagat 2100
cacatcgact ggggtgaatt tcaggtcgat ggaagatctg cacgcggagg tcaacagacg 2160
gctaatgatg ctgcagcaga ggcattaaat gcaggtaatg cattagaagc tctgcagata 2220
ataagggaga aactcccaga aaaatatatt tttcagtatc acaacctcaa acctaactta 2280
gaggctattt ttcttcctcc tccagatctt tatcaaccac cttttcctct ttcttctttc 2340
actagagttc cagaaattat tcaagagtgg gccgattctt attttggttt ggatcccgct 2400
gcgcggcctt ttagatataa tagtttaatc atagagggtg attcaagaac tggtaaaaca 2460
atgtgggcca ggtgtttagg tccacataat tatattactg gtcatttaga ttttagttta 2520
aaaacttata atgataatgt tctgtataac gtcattgatg acgtagatcc caattactta 2580
aagatgaagc attggaagca ccttataggc gcacaaagag agtggcagac aaacttaaag 2640
tatggaaaac cacgtgtcat taaaggtggt attcccagta ttatattatg caatccaggc 2700
gaaggcagct cttaccagga cttcctcaat aaatcagaaa atgaagccct taggtcctgg 2760
acattacaaa attcagtctt cgccaaactc acaagtcctc tctttgataa caatcaagaa 2820
gcgtcctcgc aagatcaaac ttccttgtaa gtgtcatttc acaattcatc atgaatgtaa 2880
tcagggattt tcgcacagag gaacccatca cgctgcaaca agcgacg 2927

## Description

### Field of the invention

The present invention belongs to the field of plant genetic engineering, and relates to a method for improving ability to resist against intrusive DNA viruses of plant.

### Technical Background

DNA viruses exist widely in the nature and have a great number of hosts in bacteria, animals and plants. Most known DNA viruses will cause severe infectious diseases and consequently result in significant loss in economic crops and mammals (including human). For example, geminivirus is a family of single-stranded DNA viruses in plants, which is the only type of virus with geminate particles. It is the largest known family of single-stranded DNA viruses. The genome of geminivirus can either be a single component or two components, with the size of about 2.5-3.1kb. Geminiviruses are transmitted by insects, and most of them infect the phloem of the plant. Currently, it has been reported that geminiviruses have caused enormous loss in the crops such as tomato, cassava and cotton. From 1991 to 1992, this virus caused a loss of 140 million dollars in tomato production in Florida, US. From 1992-1997, cotton leaf curl virus resulted in a loss of 5 billion dollars in Pakistan. For cassava, a loss of 1 billion pounds was caused each year in the world. In 1999, Science published a special report "Geminiviruses Emerge as Serious Crop Threat" to elucidate the hazardness of geminiviruses.

Conventional methods for resisting geminiviruses mainly focus on blocking the replication of DNA virus or interfering with the expression of pathogenic proteins thereof. The replication mechanism of geminivirus is relatively conserved. After invasion into the host cells, a replication-associated protein will be expressed by the viral DNA. The protein then binds to the origin of replication in the virus genome and initiates the replication of the DNA virus. When the single-stranded DNA of geminivirus enters into the host cell, a complementary strand will be synthesized in accordance to the single-stranded DNA, and a double-stranded intermediate (dsDNA) will be formed. The virus only encodes a replication-associated protein (Rep) and a replication enhancer protein (Ren). The Rep protein binds to the origin of replication in the double-stranded DNA and generates a nick at the conserved sequence TAATATTAC so as to start the rolling circle replication. Therefore, by introducing a full-length or partial exogenous Rep protein to compete with the endogenous Rep protein, the replication of the geminivirus can be inhibited. However, use of this technology is limited by the disadvantages such as high expression, interfering with the growth of the host cells, and non-universality. There is another report that virus resistance is achieved by using RNAi to interfere with the expression of the pathogenic protein of the virus. This technique is not universal either as it is homology-dependent. In 2005, Takashi Sera developed a method for inhibiting replication of geminivirus on the basis that Zinc finger binding proteins specifically bind to a double-stranded DNA. Beet severe curly top virus (BSCTV) was used as a model. An exogenous artificial zinc finger protein (AZP) was introduced to compete with the viral replication initiating protein Rep for binding to the origin of replication of the double-stranded intermediate so as to prevent the replication of the virus. However, this method only works for those geminiviruses having Rep as the replication initiating protein, and thus cannot bring about broad spectrum resistance to DNA viruses.

Recently, it has been found that there is an adaptive immune response system in bacteria which can specifically remove intrusive double-stranded DNA fragment, such as phages and plasmids. Such immune system is composed of Clustered Regularly Interspaced Short Palindromic Repeat sequences (CRISPR), trans-acting CRISPR RNA (tracrRNA) and CRISPR-associated genes (Cas gene). Some viral DNA fragments will be recorded within the CRISPR repeat sequences and then a CRISPR RNA comprising the viral sequences can be expressed. When the virus invades the cell again, the CRISPR RNA, with the aid of a tracrRNA, will guide the endonuclease encoded by the Cas gene to recognize and clear the exogenous double-stranded viral DNA.

According the differences between the sequences of core elements of the Cas genes, CRISPR/Cas immune systems can be divided into three types: type I, type II and type III. Type I and III require multiple proteins encoded by Cas genes to form a complex for cleaving the double-stranded DNA, while type II only need a Cas9 protein. Therefore, type II CRISPR/Cas system is most widely used. Recent studies found that crRNA and tracrRNA can be fused into a single-guide RNA (sgRNA) by artificial synthesis. Such sgRNA is capable of guiding Cas9 endonuclease to cleave DNA at a target site.

The virus resistance of eukaryotes would be significantly improved if a similar CRISPR/Cas system can be introduced into eukaryotes.

### Summary of the Invention

One object of the invention is to provide a method for making a plant with improved ability to resist against a DNA virus.

The method of the invention for making a plant with improved ability to resist against a DNA virus specifically can comprises the following steps:
(1) selecting a number of target sequences from the genomic sequence of the DNA virus; designing and synthesizing DNA fragments which are reverse-complement to said target sequences respectively;
   wherein said target sequence has the sequence of 5'-N_{X}-NGG-3' 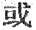 5'-CCN-N_{X}-3';
   N represents any one of A, G, C and T; 14≤X≤30, and X is an integer; N_{X} represents X contiguous deoxyribonucleotides;
(2) constructing said several DNA fragments obtained in step (1) into a vector for expressing CRISPR/Cas9 nuclease so as to obtain several recombinant vectors;
   wherein said recombinant vectors are capable of transcribing a guide RNA and expressing a Cas9 protein; said guide RNA is a RNA with a palindromic structure formed by base pairing between a crRNA and a tracrRNA; said crRNA contains a RNA fragment transcribed from said DNA fragment;
(3) introducing said several recombinant vectors into recipient plants respectively; obtaining a plant with improved ability to resist against the DNA virus from said recipient plants;
   wherein said DNA virus is a double-stranded DNA virus or a single-stranded DNA virus with a double-stranded DNA as an intermediate.

The present invention also provides a method for making a plant with improved ability to resist against a DNA virus, which specifically comprises the following steps:
1) selecting a target sequence from the genomic sequence of the DNA virus, wherein said target sequence has the sequence of 5'-N_{X}-NGG-3' 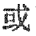 5'-CCN-N_{X}-3';
   N represents any one of A, G, C and T; 14≤X≤30, and X is an integer; N_{X} represents X contiguous deoxyribonucleotides;
2) constructing a recombinant vector for expressing CRISPR/Cas9 nuclease, wherein said recombinant vector is capable of transcribing a guide RNA that specifically targets the target sequence of step 1), and expressing a Cas9 protein;
3) introducing said several recombinant vectors into a plant, thereby a plant with improved ability to resist against a DNA virus is obtained.

In some embodiments, said DNA virus is a double-stranded DNA virus or a single-stranded DNA virus with a double-stranded DNA as an intermediate. Said recombinant vector for expressing CRISPR/Cas9 nuclease is capable of transcribing a guide RNA and expressing a Cas9 protein. Said guide RNA is a RNA with a palindromic structure formed by base pairing between a crRNA and a tracrRNA.

Another object of the invention is to provide a method for improving the ability of a plant to resist against a DNA virus.

The method of the invention for improving the ability of a plant to resist against a DNA virus specifically can comprise the following steps:
(a) obtaining a target DNA fragment according to a method comprising the following steps:
   (a1) selecting a number of target sequences from the genomic sequence of the DNA virus to be resist; designing and synthesizing DNA fragments which are reverse-complement to said target sequences respectively;
      wherein said target sequence has the sequence of 5'-N_{X}-NGG-3' 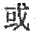 5'-CCN-N_{X}-3';
      N represents any one of A, G, C and T; 14≤X≤30, and X is an integer; N_{X} represents X contiguous deoxyribonucleotides;
   (a2) constructing said several DNA fragments obtained in step (a1) into a vector for expressing CRISPR/Cas9 nuclease so as to obtain several recombinant vectors;
      wherein said recombinant vectors are capable of transcribing a guide RNA and expressing a Cas9 protein; said guide RNA is a RNA with a palindromic structure formed by base pairing between a crRNA and a tracrRNA; said crRNA contains a RNA fragment transcribed from said DNA fragment;
   (a3) introducing said several recombinant vectors into recipient plants (1) respectively; obtaining a plant with improved ability to resist against the DNA virus from said recipient plants (1), which is designated as the target plant;
      identifying the DNA fragment in the recombinant vector introduced into said target plant as the target DNA fragment;
(b) constructing said target DNA fragment into a vector for expressing CRISPR/Cas9 nuclease and introducing said recombinant vector as obtained into a recipient plant (2) according to the steps (a2) and (a3), so as to improve the ability of the recipient plant (2) to resist against the DNA virus;
   said DNA virus is a double-stranded DNA virus or a single-stranded DNA virus with a double-stranded DNA as an intermediate,
   said recipient plant (1) and said recipient plant (2) can be the same or different.

In the above mentioned methods, "obtaining a plant with improved ability to resist against the DNA virus from said recipient plants (recipient plants (1))" is achieved by a method comprising the following steps:
(I) in step (3), introducing an empty vector into a recipient plant (or recipient plant (1)) as a control;
   wherein said empty vector is the vector for expressing CRISPR/Cas9 nuclease without insertion of said DNA fragments;
(II) introducing a vector for expressing said DNA virus into a number of recipient plants (or recipient plants (1)) carrying the recombinant vectors, so as to obtain a number of plants (A); introducing an expression vector for expressing said DNA virus into the recipient plant carrying the empty vector, so as to obtain a plant (B); detecting the DNA virus content in said plants (A) and said plant (B); selecting a plant with significantly (P<0.05) lower DNA virus content than plant (B) from the plants (A), so as to obtain a plant A'; the recipient plant (or recipient plant (1)) carrying the recombinant vector corresponding to said plant A' is the plant with improved ability to resist against the DNA virus.

Said step of "selecting a plant with significantly lower DNA virus content than said plant (B) from the plants (A)" preferably comprises selecting a plant with significantly (P<0.01) lower DNA virus content than plant (B) from the plants (A), or preferably comprises selecting a plant without any disease phenotype from the plants (A)".

In steps (2) and (a2) of said methods, the RNA fragment is capable of complementarily binding to the target fragment. The target fragment is a sequence within the genome of a double-stranded DNA virus or within the double-stranded DNA intermediate of a single-stranded DNA virus, which corresponds to the sequence "Nx" in step (1).

In step (b) of said methods, the guide RNA is transcribed and the Cas9 protein is expressed by the recombinant vector in the recipient plant (2). The CRISPR/Cas9 nuclease formed by the guide RNA and the Cas9 protein is capable of inhibiting the replication of the DNA virus in the recipient plant (2) and thereby improving the ability of the recipient plant (2) to resist against the DNA virus. In particular, the CRISPR/Cas9 nuclease formed by the guide RNA and the Cas9 protein cleaves the target fragment so as to inhibit the replication of the DNA virus in the recipient plant (2).

In a specific embodiment of the invention, the vector for expressing CRISPR/Cas9 nuclease is the pHSN401 vector. Correspondingly, the recombinant vector is a recombinant plasmid obtained by inserting the DNA fragment into the two Bsa I sites of the pHSN401 vector in a forward direction.

In a specific embodiment of the invention, said X is 20.

In a specific embodiment of the invention, said plant is a dicotyledon.

In a specific embodiment of the invention, said plant is tobacco, such as *Nicotiana benthamiana*.

In one embodiment of the invention, said DNA virus is a geminivirus, in particular, a beet severe curly top virus (BSCTV). Correspondingly, the DNA fragment designed in accordance to the target sequence (table 1) is any one of the sequences listed in table 2. Said target DNA fragment is any one of the sequences listed in table 2 (i.e., the complementary sequence of any one of SEQ ID NOs:1-15). Preferably, Said target DNA fragment is any one of the sequences listed in table 2 excluding V4 and V9 (i.e., the reverse-complement sequence of any one of SEQ ID NOs:1-3, 4-8 and 10-15). More preferably, Said target fragment is V4 or V9 (i.e., the reverse-complement sequence of SEQ ID NO:7 or 8). Said expression vector (i.e., the "expression vector for expressing said DNA virus" above) is the plasmid pCambiaBSCTV1.8. The plasmid pCambiaBSCTV1.8 is obtained by a method comprising the following steps: (a1) inserting a DNA fragment of SEQ ID NO:16 between EcoRI and BamHI of the pCambia1300 vector in the forward direction so as to obtain an intermediate vector; (a2) inserting a DNA fragment of SEQ ID NO:17 into the EcoRI site of said intermediate vector, thereby the plasmid pCambiaBSCTV1.8 is obtained.

The method of the invention mimics the immune system of bacteria. A sgRNA that specifically recognizes a target site in the virus is expressed in the plant so as to induce a Cas9-mediated clearance of double-stranded viral DNA in the plant. Using beet severe curly top virus (BSCTV) as a model virus, the present invention for the first time shows an effective inhibition of replication of BSCTV in a plant with the CRISPR/CAS9 system. The method of the invention merely depends on the genomic sequence of the virus, without the need of knowing specific functions of viral genes. Therefore, the method can be widely used for resisting against various known double-stranded DNA viruses or single-stranded viruses with double-stranded DNA as an intermediate.

### Brief Description of the Drawings

Figure 1 shows relative contents of BSCTV in various groups of tobacco plants. The relative content of BSCTV in pHSN401 empty vector control is represented as 1. The relative contents of BSCTV in other groups are represented as a ratio to the pHSN401 empty vector control.
Figure 2 shows the phenotypes of the tobacco plants in various groups. A is the pHSN401 empty vector control; B is the V7 group; C is the V8 group; D is the V4 group.

### Detailed Embodiments

The experimental methods used in the following Examples are all conventional methods, unless otherwise indicated.

The materials, reagents used in the following Examples are all commercially available, unless otherwise indicated.

The pHSN401 vector is described in "Hui-Li Xing et al., A CRISPR/Cas9 toolkit for multiplex genome editing in plants. BMC plant biology 2014", and can be obtained from the Institute of Genetics and Developmental Biology of the Chinese Academy of Sciences.

*Nicotiana benthamiana* is described in "Hai-tao Cui et al., Establishment of a tissue culture and genetic transformation system for Nicotiana benthamiana, Science In Shandong, 2006, Volume 01", and can be obtained from the Institute of Genetics and Developmental Biology of the Chinese Academy of Sciences.

pCambia1300 vector is obtained from Youbio Co. Ltd. (CAT.No.: VT1375).

Agrobacterium strain EHA105 is obtained from Youbio Ltd. (CAT.No.: ST1140).

### Example 1. Establishment of a method for improving the ability of plant to resist DNA virus

In this example, beet severe curly top virus (BSCTV) was chosen as the DNA virus to be resisted, while tobacco (*Nicotiana benthamiana*) was used the target plant of BSCTV, so as to establish a method for improving the ability of a plant to resist against a DNA virus.

BSCTV is a single stranded DNA virus. However, its amplification requires the rolling circle replication. Firstly, a complementary strand is synthesized with the single-stranded viral DNA as the template to generate a double-stranded circular intermediate. Then, a nick is generated on the double-stranded intermediate, and a great amount of single-stranded viral DNA is produced with the complementary strand as the template. Therefore, the double-stranded intermediate plays an important role in the amplification of virus genomic DNA. The present inventors designed several sgRNA specific to the double-stranded intermediate of the virus for directing the Cas9 to remove the double-stranded intermediate of BSCTV.

### I. Construction of a Crispr/Cas9 system binary vector for dicots

1. A structural map of beet severe curly top virus (BSCTV) was obtained based on the results of next generation sequencing (SEQ ID NOs: 16 and 17).
2. A region in the structural map was selected randomly and named V region. Target fragments (Table 1) were designed to cover the V region which is 300bp in length. In addition, a target fragment of a non-viral sequence is used as control.

**Table 1. Design of V region target fragments**

| Region | No. | Sequence |
|---|---|---|
| | V1 | TCATTGGATTATCATAGACAAGG (SEQ ID NO:1) |
| | V2 | ATTATCATAGACAAGGATGTTGG (SEQ ID NO:2) |
| | V3 | CCTACTAAGTTATCGAGTATATT (SEQ ID NO:3) |
| | V4 | TGATATTCCCGATAACGGTCAGG (SEQ ID NO:4) |
| | V5 | CCGTCTACTTATCGTATTCGAAG (SEQ ID NO:5) |
| | V6 | TTCGAAGAGATATGAACGAGAGG (SEQ ID NO:6) |
| | V7 | GGTTTATTGTGAAGAAGAAATGG (SEQ ID NO:7) |
| V | V8 | AGAACTCATTTGATGTCTACTGG (SEQ ID NO:8) |
| | V9 | TGGTACTGGATATGGAGGGAAGG (SEQ ID NO:9) |
| | V10 | TGGAGGGAAGGAGACTTACAAGG (SEQ ID NO:10) |
| | V11 | CCTTCAATGCCAAATTACAAGAA (SEQ ID NO:11) |
| | V12 | CCGATGAATATCAATGTCCGCAA (SEQ ID NO:12) |
| | V13 | ATCTGAACATGAGGACTATTTGG (SEQ ID NO:13) |
| | V14 | ACTATTTGGAAAGACACCGGTGG (SEQ ID NO:14) |
| | V15 | TGGGAAGTATGAAGACGTGAAGG (SEQ ID NO:15) |

Underlined nucleotides in Table 1 represent PAM sequence.
3. Single-stranded oligonucleotides with sticky ends (Table 2) were synthesized according to the target fragments designed in step 2. The double-stranded DNA with sticky ends formed by annealing of the oligonucleotides was inserted between the two BsaI restriction sites of the pHSN401 vector in a forward direction to obtain a recombinant plasmid. The positive recombinant plasmid with a reverse-complement sequence of the target sequence of Table 1 inserted between the two BsaI restriction sites of the pHSN401 vector was confirmed by sequencing and designated pHSN401-sgRNA. The positive recombinant plasmid can transcribe a guide RNA (sgRNA) specific to the corresponding target fragment, and express a Cas9 protein.

**Table 2 Oligonucleotides corresponding to the V region target fragments**

| Region | No. | sequence |
|---|---|---|
| | V1 | V1F: ATTGTCATTGGATTATCATAGACA |
| V | | V1R: AAACTGTCTATGATAATCCAATGA |
| | V2 | V2F: ATTGATTATCATAGACAAGGATGT |
| | | V2R: AAACACATCCTTGTCTATGATAAT |
| | V3 | V3F: ATTGAATATACTCGATAACTTAGT |
| | | V3R: AAACACTAAGTTATCGAGTATATT |
| | V4 | V4F: ATTGTGATATTCCCGATAACGGTC |
| | | V4R: AAACGACCGTTATCGGGAATATCA |
| | V5 | V5F: ATTGCTTCGAATACGATAAGTAGA |
| | | V5R: AAACTCTACTTATCGTATTCGAAG |
| | V6 | V6F:ATTGTTCGAAGAGATATGAACGAG |
| | | V6R: AAACCTCGTTCATATCTCTTCGAA |
| | V7 | V7F:ATTGGGTTTATTGTGAAGAAGAAA |
| | | V7R: AAACTTTCTTCTTCACAATAAACC |
| | V8 | V8F: ATTGAGAACTCATTTGATGTCTAC |
| | | V8R: AAACGTAGACATCAAATGAGTTCT |
| | V9 | V9F:ATTGTGGTACTGGATATGGAGGGA |
| | | V9R: AAACTCCCTCCATATCCAGTACCA |
| | V10 | V10F:ATTGTGGAGGGAAGGAGACTTACA |
| | | V10R: AAACTGTAAGTCTCCTTCCCTCCA |
| | V11 | V11F: ATTGTTCTTGTAATTTGGCATTGA |
| | | V11R:AAACTCAATGCCAAATTACAAGAA |
| | V12 | V12F: ATTGTTGCGGACATTGATATTCAT |
| | | V12R: AAACATGAATATCAATGTCCGCAA |
| | V13 | V13F: ATTGATCTGAACATGAGGACTATT |
| | | V13R: AAACAATAGTCCTCATGTTCAGAT |
| | V14 | V14F: ATTGACTATTTGGAAAGACACCGG |
| | | V14R: AAACCCGGTGTCTTTCCAAATAGT |
| | V15 | V15F:ATTGTGGGAAGTATGAAGACGTGA |
| | | V15R: AAACTCACGTCTTCATACTTCCCA |

### II. Screening for the active sgRNA with a tobacco transient system

For the first time, the inventors establish a tobacco transient system for screening active sgRNA with resistance to BSCTV.

### 1. preparation of tobacco plants

Tobacco (*Nicotiana benthamiana*) seeds were directly sown into nutritional soil. Seedlings were transplanted after two weeks, one seedling per 9 × 9cm square. Seedlings were grown for two weeks to 8-9 leaves. Two lateral leaves of similar size were selected for injection. The growth conditions in the green house: 16h light and 8h dark; the temperature is 24°C.

### 2. Resistant vector and virus inoculation

The pCFH vector containing BSCTV was obtained from American Type Culture Collection (ATCC, http://www.lgcstandardsatcc.org/, ATCC® Number: PVMC-6™). A 0.8-copy BSCTV fragment (SEQ ID NO:16) was obtained by double digestion of the pCFH vector with EcoRI and BamHI, and inserted between the EcoRI and BamHI restriction sites of pCambia1300 vector. The resulted recombinant plasmid was designated as pCambiaBSCTV0.8. Then, a 1-copy BSCTV fragment (SEQ ID NO:17) was obtained by digestion with EcoRI, and constructed into pCambiaBSCTV0.8 at the EcoRI site in a forward direction. The resulted recombinant vector was designated as pCambiaBSCTV1.8. The method for construction of this recombinant vector was disclosed in Chen et al., BSCTV C2 Attenuates the Degradation of SAMDC1 to Suppress DNA Methylation-Mediated Gene Silencing in Arabidopsis. 2011.

As 1.8 copies of virus were integrated into the binary vector with a backbone of pCambia1300 (1.8 copies means that, addition 0.8 copy of virus sequence is necessary for the virus integrated in the binary vector to form circular individuals in the plant), the self replication of the virus can be achieved upon integration into the genome of *Nicotiana benthamiana* by the Agrobacterium injection method.

The pHSN401-sgRNA plasmid was transformed into Agrobacterium strain EHA105 with the liquid nitrogen freezing-thawing method. A single colony was picked up into 1ml LB supplemented with kanamycin and rifampicin and incubated with shaking overnight. Then the bacteria were inoculated into 5ml LB supplemented with kanamycin and rifampicin and incubated with shaking overnight. The culture was centrifuged at 3600rpm for 10 min, resuspended with 10mM MgCl₂ and diluted to OD600=1.5. 0.1% (v/v) 200mM acetosyringone (As) was added. The solution was kept still for two hours and injected into the two tobacco leaves as prepared above.

Two days later, pCambiaBSCTV1.8 plasmid was transformed into Agrobacterium strain EHA105 with the same method. The scaled up culture was resuspended with 10mM MgCl₂, diluted to OD600=0.5 and injected to the two leaves that already injected with the pHSN401-sgRNA plasmid.

The pHSN401 plasmid was used as the control in the experiment. At least one tobacco plant was used in each experiment.

10 days later, the phenotypes of the tobacco plants in various groups were recorded. The injected leaves were sampled and ground into powder in liquid nitrogen. 100mg of each sample was taken for DNA extraction using TIANGEN DNA quick Plant System (no centrifuge column). PPR (Pentatricopeptide repeat containing protein) gene (Accession number: GO602734) of *Nicotiana benthamiana* was selected as the internal standard, and an appropriate fragment of 133bp was selected for amplification with the primers:
PPR-F: 5 '-CTCGGCCAAGAAGATCAACCATAC-3';
PPR-R: 5 '-GGTGCTTTATGTGGTTGTAGTTATGC-3'.

The BSCTV fragment to be amplified is 76bp, and the primers are
BSCTV-F: 5'-CAGGGATTTTCGCACAGAGGAAC -3';
BSCTV-R: 5'-GATTCGGTACCAAGTCCACGGG -3'.

The reagent for qPCR is Roche Lightcycler @480 SYBR Green I Master. The reaction system of the qPCR: 2×mix 10µl; primers(each) 1µl; ddH₂O 4µl; DNA template 4µl.

The BSCTV contents in tobacco plants of the experiment groups relative to the empty vector control were calculated according to the 2-^{ΔΔCT} method:
(1) the BSCTV content of each group was normalized:
   virus content=2^{(CT (internal PPR) -CT (BSCTV))}
(2) the virus content of the experiment group relative to the control group was virus contentₑₓₚₑᵣᵢₘₑₙₜ/virus content_{control}. Results were statistically analyzed in Excel.

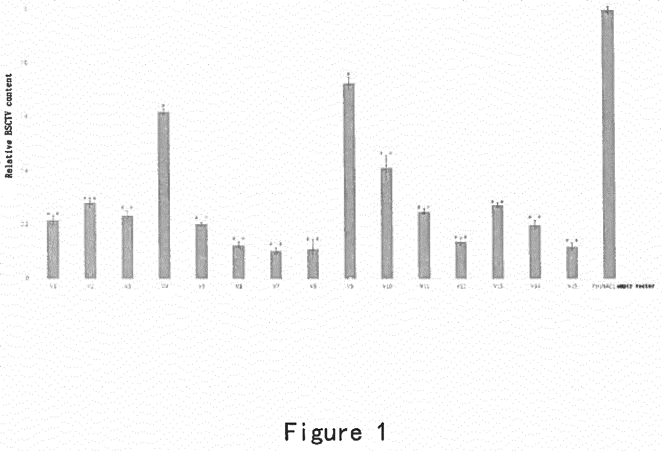

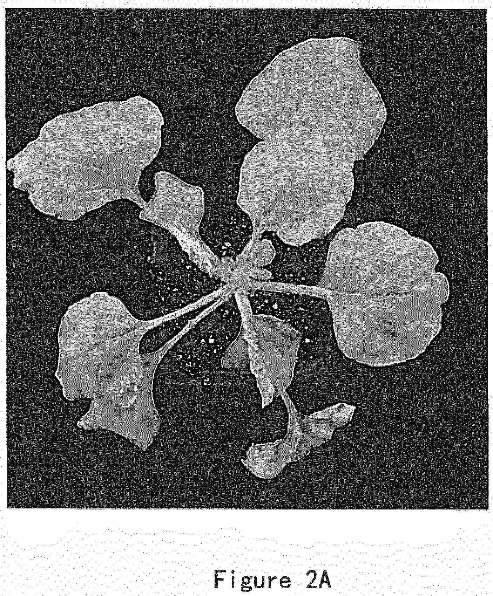

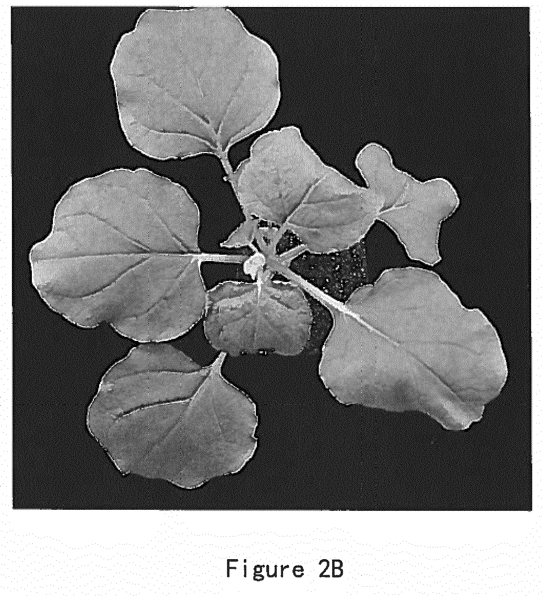

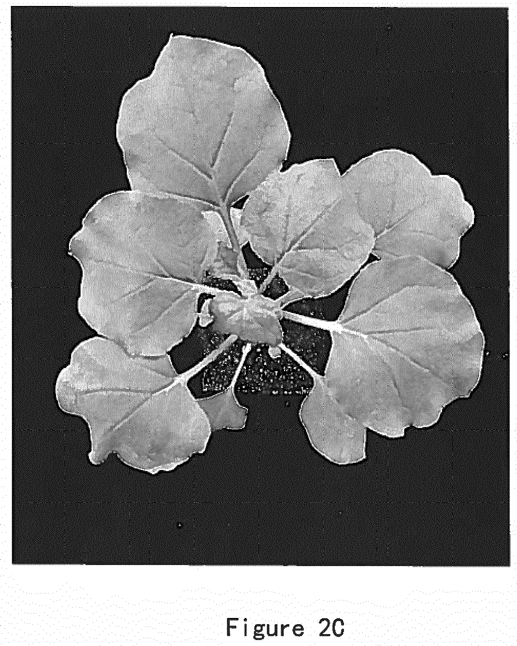

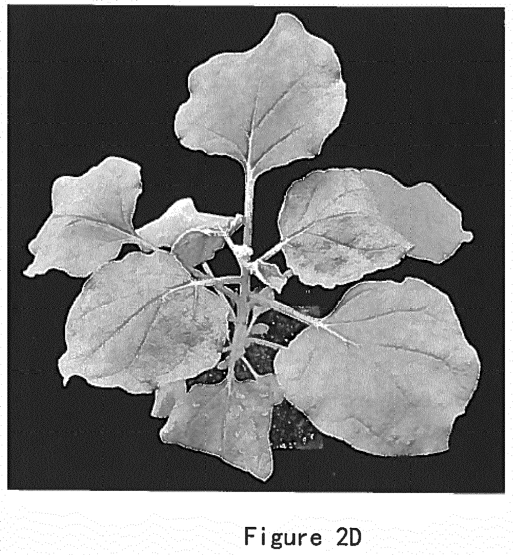

The relative BSCTV content in each group of tobacco plants was shown in Figure 1. It can be seen that, the relative BSCTV contents in the experiment groups are lower as compared with the pHSN401 control. Upon statistical analysis, all the experiment groups have significant difference at P<0.01 level compared with the empty vector control, except for V4 and V9, which have a significance level of P<0.05. The tobacco plants in the pHSN401 control group exhibited clear BSCTC infection symptoms. The V4 and V9 plants also showed clear BSCTC infection symptoms, although better than the control plants. The V7 and V8 plants with the most significant difference showed normal phenotypes, exhibiting strong ability to resist against the virus. It was found that the relative content of BSCTV in the plants is consistent to the disease phenotype (tobacco plants with representative phenotypes were shown in Figure 2).

## Claims

1. A method for making a plant with improved ability to resist against a DNA virus, comprising the following steps:
(1) selecting a number of target sequences from the genomic sequence of the DNA virus to be resist; designing and synthesizing DNA fragments reverse-complement to said target sequences respectively;
wherein said target sequences have the sequence of 5'-N_{X}-NGG-3' 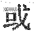 5'-CCN-N_{X}-3';
N represents any one of A, G, C and T; 14≤X≤30, and X is an integer; N_{X} represents X contiguous deoxyribonucleotides;
(2) constructing said DNA fragments obtained in step (1) into a vector for expressing CRISPR/Cas9 nuclease so as to obtain a number of recombinant vectors;
wherein said recombinant vectors are capable of transcribing a guide RNA and expressing a Cas9 protein; said guide RNA is a RNA with a palindromic structure formed by base pairing between a crRNA and a tracrRNA; said crRNA contains a RNA fragment transcribed from said DNA fragment;
(3) introducing said recombinant vectors into recipient plants respectively; obtaining a plant with improved ability to resist against the DNA virus from said recipient plants;
said DNA virus is a double-stranded DNA virus or a single-stranded DNA virus with a double-stranded DNA as an intermediate.

2. A method for improving the ability of a plant to resist against a DNA virus, comprising the following steps:
(a) obtaining a target DNA fragment according to a method comprising the following steps:
(a1) selecting a number of target sequences from the genomic sequence of the DNA virus to be resist; designing and synthesizing DNA fragments reverse-complement to said target sequences respectively;
wherein said target sequences have the sequence of 5'-N_{X}-NGG-3' or 5'-CCN-N_{X}-3';
N represents any one of A, G, C and T; 14≤X≤30, and X is an integer; N_{X} represents X contiguous deoxyribonucleotides;
(a2) constructing said DNA fragments obtained in step (a1) into a vector for expressing CRISPR/Cas9 nuclease so as to obtain a number of recombinant vectors;
wherein said recombinant vectors are capable of transcribing a guide RNA and expressing a Cas9 protein; said guide RNA is a RNA with a palindromic structure formed by base pairing between a crRNA and a tracrRNA; said crRNA contains a RNA fragment transcribed from said DNA fragment;
(a3) introducing said recombinant vectors into recipient plants (1) respectively; obtaining a plant with improved ability to resist against the DNA virus from said recipient plants (1), which is designated as the target plant;
identifying the DNA fragment in the recombinant vector introduced into said target plant as the target DNA fragment;
(b) constructing said target DNA fragment into a vector for expressing CRISPR/Cas9 nuclease and introducing the resultant recombinant vector into a recipient plant (2) according to the steps (a2) and (a3), so as to improve the ability of the recipient plant (2) to resist against the DNA virus;
wherein said DNA virus is a double-stranded DNA virus or a single-stranded DNA virus with a double-stranded DNA as an intermediate,
said recipient plant (1) and said recipient plant (2) can be the same or different.

3. The method according to claim 1 or 2, wherein said vector for expressing CRISPR/Cas9 nuclease is a pHSN401 vector.

4. The method according to claim 3, wherein the recombinant vector is a recombinant plasmid obtained by inserting the DNA fragment between the two Bsa I sites of the pHSN401 vector.

5. The method according to any one of claims 1-4, wherein X is 20.

6. The method according to any one of claims 1-5, wherein the plant is a monocotyledon or a dicotyledon.

7. The method according to claim 6, wherein the dicotyledon is tobacco.

8. The method according to any one of claims 1-7, wherein said DNA virus is a geminivirus.

9. The method according to claim 8, wherein the geminivirus is beet severe curly top virus.
